# EUROPEAN PATENT SPECIFICATION

(11) **EP 0 414 297 B1**
(45) Date of publication and mention of the grant of the patent: **16.10.1996**
(21) Application number: 90202109.6
(22) Date of filing: 02.08.1990
(51) Int. Cl.: C12N 15/57, C12N 9/54, C12N 15/75, C12N 1/21

(54) **Efficient production of mutant proteases**
Ergiebige Herstellung von Protease-Mutanten
Production effective de protéases mutantes

(30) Priority: 11.08.1989 EP 89202117
(43) Date of publication of application: 27.02.1991
(73) Proprietor: GIST-BROCADES N.V., NL-2600 MA Delft (NL)
(72) Inventor: Van der laan, Johannes Cornelis, NL-1062 CP Amsterdam (NL); Van Eekelen, Christiaan Albertus Gerardus, NL-2661 HD Bergschenhoek (NL)
(74) Representative: Visser-Luirink, Gesina, Dr.

(56) References cited:
- EP-A- 0 130 756
- EP-A- 0 283 075
- EP-A- 0 284 126
- EP-A- 0 328 229
- WO-A-86/01825
- COMUN. JORN. COM. ESP. DETERG., vol. 19, 1988, pages 257-266; J.H. VAN EE et al.: "Protein-engineering of the high alkaline detergent protease Maxacal"
- IDEM

## Description

### Technical Field

The present invention relates to a method for the production of mutant proteins using recombinant DNA techniques. More specifically, the invention relates to the efficient production of protein engineered Bacillus proteases using a homologous Bacillus host strain, from which the capacity to produce the corresponding unmodified protease has been eliminated.

### Background of the Invention

Bacilli are widely used for the production of industrially important enzymes such as α-amylases, neutral proteases and alkaline (or serine) proteases, see, for example, Debabov: "The industrial use of Bacilli" in: The Molecular Biology of Bacilli, Acad. Press, New York, 1982.

The main advantages of using Bacilli are the large quantities of protein secreted, their lack of production of hazardous substances and their long history of safe industrial use. Several species of Bacilli have therefore been approved for the production of proteins destined for food applications.

Palva et al., Gene 19 (1982) 81-87, demonstrated the feasibility of Bacillus subtilis as an expression host for heterologous proteins, by performing the expression of the α-amylase gene from B. amyloliquefaciens in B. subtilis. Other references describing heterologous gene expression in B. subtilis are, e.g., Fahnestock and Fisher, J. Bacteriol. 165 (1986) 796-804, who showed the expression of the Staphylococcus aureus Protein A gene; Schein et al., Biotechnology 4 (1986) 719-725, disclosing the expression of human interferon-α2; and Wang et al., Gene 69 (1988) 39-47, showing the expression and secretion of human atrial natriuretic α-factor. To ensure the proper secretion of these heterologous proteins the former two references used the α-amylase signal sequence and the latter reference the signal sequence of the B. subtilis subtilisin gene (aprE) fused to the gene to be expressed.

A major problem of Bacillus strains as production hosts for recombinant proteins is that they produce and secrete a variety of proteases which tend to degrade the heterologous proteins produced. See, e.g., Old and Primrose, "Principles of Gene Manipulation", 3rd ed. (1985) 289, Blackwell, Oxford.

In International Patent Application (PCT) WO 89/04866 the important of extracellular proteases is pointed out; about 90% of the proteolytic activity is attributed to the neutral metalloprotease (npr) and the serine protease (alkaline protease = apr).

Various solutions have been proposed to overcome this degradation problem.

Kawamura and Doi, J. Bacteriol. 160 (1984) 442-444, disclose a B. subtilis double mutant deficient in both extra-cellular neutral and alkaline protease. First B. subtilis DB100 was made neutral protease negative by transferring the nprR2 and the nprE18 mutations from B. subtilis NT18. Subsequently, a 178 bp fragment containing a part of the apr gene was deleted by gene conversion.

Fahnestock and Fisher, Appl. Environ. Microbiol. 53 (1987) 379-384, describe the construction of an apr negative B. subtilis strain starting from a npr negative strain. They used a plasmid vector containing the apr gene and introduced the cat gene (conferring chloramphenicol resistance) both as an inactivator and as a selectable marker. Subsequently, the chromosomal apr gene was replaced by this construct.

Sloma et al., J. Bacteriol. 170 (1988) 5557-5563, detected and sequenced a third extracellular protease gene (epr) from B. subtilis. Partial deletion of this gene showed that it ontributed only marginally to the extracellular protease activity.

Upon closer inspection of the produced strains it appears that none of the referenced B. subtilis strains was completely devoid of extracellular proteolytic activity. Therefore, there is still a need for improved protease deficient Bacillus strains, which can be used in the production of heterologous proteins. This need is even stronger when the aim is the expression of mutated proteases.

In several publications the production of mutant proteases is described using Bacillus subtilis host strains which are incapable of expressing the wild-type protease gene, thus avoiding mixtures of mutant and wild-type proteases.

In EP-A-0246678 (the contents of this applicaton correspond with EP-A-0130756) a B. subtilis strain is disclosed which is incapable of secreting enzymatically active subtilisin or neutral protease, but which expresses heterologous protease genes isolated or derived from B. amyloliquefaciens. Site-specific saturation mutagenesis was performed on the B. amyloliquefaciens gene and the mutants were expressed. The negative background of the B. subtilis host strains was obtained by partial deletion of either or both of the original protease genes, or by N-methyl-N′-nitro-N-nitrosoguanidine (NTG) mutagenesis. It was further stressed that the B. subtilis host described is normally sporulating and that asporogenous mutants are unsatisfactory for the production of (recombinant) proteins.

In WO 86/01825 (see Fahnestock and Fisher, cited above) Bacillus strains are described, in particular B. subtilis, with reduced extracellular protease levels. The alkaline protease gene is inactivated by in vitro insertion of a gene coding for a protein which confers a phenotypic trait on the microorganism. The plasmid vector containing the insertionally inactivated protease gene is transformed to B. subtilis and the inactivated gene replaces the native chromosomal gene by homologous recombination.

WO 88/08033 discloses subtilisin analogs from subtilisin Carlsberg, subtilisin DY, subtilisin BPN′, an apr subtilisin from B. subtilis and a subtilisin from B. mesentericus. These mutants are obtained by replacing one or more amino acids in or near the calcium binding site with negatively charged amino acids (e.g. Asp or Glu). The mutants are expressed in B. subtilis.

WO 89/04866 discloses a B. subtilis strain with low secreted protease activity, which was made by introducing a spoOH mutation in an apr⁻npr⁻ double mutant, thereby making the strain asporogenous. The obtained triple mutant apr⁻npr⁻ spoOH⁻ showed very low secreted protease activity.

The references discussed.above describe the expression of homologous and heterologous genes in B. subtilis and the construction of protease-negative B. subtilis strains. It has appeared that protease-negative strains made by partial deletion of the protease gene or by insertion of another gene in the protease gene show several drawbacks, e.g.:
- the original gene may be reactivated. If a gene which is homologous to the inactivated chromosomal gene is introduced into the cell (e.g. on a plasmid), homologous recombination can lead to reactivation of the gene.
- the original gene, although inactivated, may lead to the production of partial expression products, when the promoter region is still active. This is disadvantageous in terms of metabolic efficiency.
- the introduction of unwanted phenotypical traits, e.g. antibiotic resistance, depending on the inserted gene.
- plasmid instability, due to homology between a genomic sequence and a part of the plasmid.

It is therefore desirable to delete all sequences with possible homology between the chromosome and the plasmid.

There are also other drawbacks for the production of alkaline proteases, or other enzymes, in B. subtilis. Although Wells et al., Nucl. Acids Res. 11, (1983) 7911-7925 show that expression of B. amyloliquefaciens subtilisin BPN′ in B. subtilis is possible with its own transcription signals, Jacobs et al., Nucl. Acids Res. 13 (1985) 8913-8926 show that such a strategy is not generally applicable. It was demonstrated that the 5′ region of subtilisin Carlsberg gene derived from B. licheniformis did not contain signals functional for transcription in B. subtilis. To achieve considerable production it was needed to insert a B. subtilis promoter in front of the gene in question. It is also suggested that not only transcriptional but also translational, secretion or maturation processes may occur less efficient in heterologous hosts, due to a not perfect Shine Dalgarno sequence or even incompatibility of the gene product with the heterologous host.

The expression problems described above can be avoided when the product is synthesized in a homologous Bacillus strain, of which high production capabilities are proven, with high efficiency resulting in high production levels.

### SUMMARY OF THE INVENTION

The present invention provides a method for the production of proteases differing in at least one amino acid from the wild-type protease, said method comprising the use of a homologous alkalophilic Bacillus strain as expression host said strain being unable to produce the wild-type protease.

In one aspect of the present invention the high alkaline protease gene is deleted from the chromosome, the obtained protease negative strain can be used as a host for the expression of homologous or heterologous proteases.

In another aspect of the present invention the chromosomal high alkaline protease gene is replaced by a mutated copy of this gene.

Also provided are methods for constructing the new transformed strains, vectors used therefor and a method of producing mutant proteases.

In a preferred embodiment mutant proteases are produced which show close homology with the proteases originally produced by the corresponding strain.

Preferred strains are Bacillus novo species PB92, derivatives thereof and closely related strains, transformed by a mutated protease encoding gene. Said gene shows at least 30%, preferably at least 50% and more preferably at least 80% homology with the wild-type gene of Bacillus PB92 encoding serine protease. The gene is preferably derived from a wild-type gene of an alkalophilic Bacillus strain. Especially preferred strains are asporogenous alkalophilic Bacilli.

### BRIEF DESCRIPTION OF THE DRAWINGS

Figure 1 shows the construction of plasmid pM58 Δ containing parts of the 5′ and 3′ flanking regions of the alkaline protease gene from Bacillus novo sp. PB92.

Figure 2 schematically shows the introduction of the temperature-sensitive origin of replication from plasmid pE194neo-1 into plasmid pM58Δ, giving the plasmid pEM58 Δ.

Figure 3 shows the introduction by homologous recombination, of the plasmid pEM58 Δ into the 5′ flanking region of the protease gene of Bacillus strain PBT110.

Figure 4 shows a simplified restriction map of the regions surrounding the deleted protease gene after illegitimate recombination. Two strains were obtained, PBT125 and PBT126.

Figure 5 nucleotide sequence of the HindIII fragments of PBT125 (A) and PBT126 (B).

Figure 6 schematic representation of the introduction of the protease gene into M13 mp18.

Figure 7 shows the nucleotide sequence of plasmid pBHA-1.

Figure 8 shows the introduction of the protease gene of Bacillus strain PBT110 into plasmid pBHA1.

Figure 9 shows the reorientation of the F1 ori containing BamHI fragment of pBHA1-MXL giving plasmid pBHAR-MXL.

Figure 10 shows the subcloning of the 3′ sequence of the high alkaline protease into plasmid pBHAR-MXL, giving plasmid pBHARB-MXL.

Figure 11 shows the deletion of the E. coli sequence from plasmid pBHARB-MXL M216Q giving plasmid pBHB-MXL M216Q.

Figure 12 shows the reorientation of the neomycin resistance gene in plasmid pE194neo-1 giving plasmid pE194neo-3.

Figure 13 schematically shows the introduction of the temperature-sensitive origin of replication from plasmid pE194neo-3 into plasmid pBHB-MXL M216Q giving plasmid pEN₃Q.

Figure 14 schematically shows the construction of strain Bacillus strain PEP111.

Figure 15 schematically shows the construction of strain Bacillus strain PEP211.

### DETAILED DESCRIPTION OF THE INVENTION

Serine proteases that are produced by strains belonging to the genus Bacillus are usually called alkaline proteases. The proteases used in the present invention are derived from alkalophilic Bacilli, and will therefore be called high alkaline proteases.

For the production of high alkaline proteases preferably alkalophilic Bacillus strains are used as host cells. For large scale production industrial strains are a need. They originate from organisms which may be isolated from the soil or are available from depositories or other sources and are obtained by genetic modification of such Bacillus strains. Industrial Bacillus strains are defined in EP-A-0134048. They are characterized by being resistant to genetic exchange, such as phage infection or transformation. The strains are stable and may or may not be capable of spore formation. They are usually prototrophic and modified to provide for high yields of endogenous protein products, such as the enzymes alpha-amylase and various proteases. The yield of an endogenous protein product obtained in an industrial production process can amount to at least 5 g/l (0.5% w/v). Industrial strains also secrete DNases, which result in degradation of DNA in the medium, providing for protection against genetic exchange.

It is advantagous to use as a host strain the strain from which the protease gene was originally isolated, since only in that case one can be sure that all the signals needed for expression are functional.

An additional advantage of using alkalophilic Bacilli as production strains is the minimal risk of contamination with other microorganisms, because of the alkaline pH during fermentation.

According to the present invention a method is disclosed for the deletion of a Bacillus protease encoding gene from the genome in such a way that reversion becomes impossible. Said deletion may be a partial deletion (as long as the sequences left in the chromosome are to short for homologous recombination with plasmid encoded protease gene) but more preferably the gene is completely deleted. The protease negative strain obtained by this method is used for the production of homologous mutant proteases after introduction of an expression vector carrying the gene encoding this mutant protease. It is, however, understood that such a protease negative strain can also be advantageously used for the expression of other proteins both homologous and heterologous. In this context we consider that the expression of mutant proteases using as a host cell, the cells where the wild-type gene was isolated from, to be homologous expression.

It has been found that by using an industrial protease producing Bacillus strain as a host for the production of the mutants of this protease, the high efficiency of the production of the original protease is transferred to the mutant protease. This results in a surprisingly superior production efficiency compared to laboratory strains. One chromosomal copy of the mutated gene gives upon expression and secretion in an industrial strain a yield even higher than 50 plasmid copies containing the mutated gene, in a laboratory strain.

It is an aspect of this invention that mutant proteases can be produced efficiently by a homologous Bacillus strain by way of exchanging the chromosomal gene or a part thereof with the corresponding mutated gene. In this way the production capacity for the wild-type protease is eliminated while at the same time introducing production capacity for a mutant protease.

As a host cell for the expression of mutated genes encoding modified or so-called protein engineered proteins it is preferable to use cells in which the genes are structurally expressed at high level.

In another aspect of the present invention it was surprisingly found that asporogenous Bacilli can be used to obtain high level expression of the protease gene.

The present invention also shows (Table 1) that to get a high production of the high alkaline protease derived from Bacillus PB92 in B. subtilis, it is preferred to insert a promoter which is active in B. subtilis, in front of the gene. If enzymes derived from other Bacilli are produced in B. subtilis insertion of a promoter active in B. subtilis or other expression signals may be necessary, which requires an extra step.

High alkaline protease producing bacilli are taxonomically not well classified and are generally referred to as alkalophilic Bacillus strains. For the present invention we define alkalophilic bacilli as Bacillus strains that grow under alkaline conditions pH 9-11 (Horikoshi, K. and T. Akiba, 1982, Alkalophilic microorganisms, Springer Verlag, New York). The alkaline proteases produced by such bacilli are also called high alkaline proteases. Examples of Bacillus strains capable of growing at alkaline pH are described in, for example, U.S. Patent Nos. 3,723,250, Re. 30,602 and 4,480,037. An example of an alkalophilic Bacillus host strain is Bacillus novo species PB92 disclosed inter alia in U.S. Patent No. Re. 30,602. Derivatives of these alkalophilic Bacillus strains, that have been optimized for protease production are employed to produce their proteases on industrial scale (See EP-A-0284126). The products are used in several industrial applications e.g. as an additive in laundry detergents. Examples of such products are Maxacal^{R} (Gist-brocades/IBIS), Savinase^{R} (NOVO), Esperase^{R} (NOVO). Mutants of such products have been described in WO 89/06279, where it is said that they are derived from Bacillus lentus strains, and in the not prepublished European Patent Application EP-A-0328229.

According to a preferred embodiment of the present invention transformed Bacillus strains are provided which produce the mutants described in EP-A-0328229 and WO 89/06279, preferably on an industrial scale.

According to the present invention a Bacillus strain is suitably used, in particular an alkalophilic Bacillus, preferably Bacillus novo species PB92. Another preferred group of Bacillus strains are the asporogenous mutants, of which PBT110 and its derivatives are most preferred. Transformation of alkalophilic Bacillus strains will preferably involve the use of protoplasts from said strains. However, the conventional protoplast transformation protocol as described by Chang, S. and S.M. Cohen, Molec. Gen. Genet. 168 (1979) 111-115, does not work for alkalophilic Bacillus strains. The protocols needed for these strains have been disclosed in EP-A-0283075, which is herein included by reference.

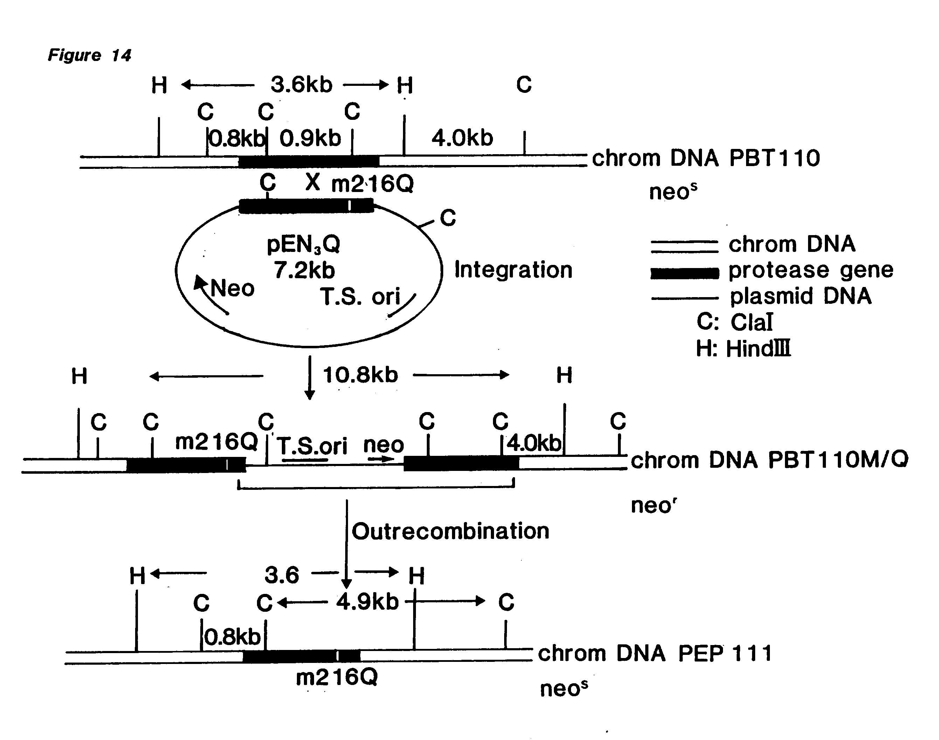

Expression of mutant protease genes in homologous hosts necessitates the replacement and/or inactivation of the wild-type gene. Some methods can be used.
A) One method is to clone the gene or part thereof, modify it by site-directed mutagenesis and reintroduce the (partial) gene into the cell on a plasmid. By homologous recombination the gene may be introduced into the chromosome. Resulting in a situation in which a wild-type and the mutant gene are tandemly located. After a second recombination the modified sequence is left in the chromosome having thereby effectively introduced the mutation into the chromosomal gene (as described in Figure 14).
B) In another method the chromosomal gene copy is inactivated by deletion. If deletion of the gene is chosen as a strategy the chromosomal gene fragment that is deleted is preferably the complete coding region. After the inactivation a mutant copy of the inactivated gene is brought into the cell on a cloning vector.

The present invention relates to Bacillus strains that are known to be efficient protease producers. In one of the preferred methods the complete high alkaline protease coding region is deleted from the chromosome. In this embodiment a vector is used containing the alkaline protease gene including its 5' and 3' regions. The protease gene is deleted from the vector in vitro, leaving behind the 5' and 3' flanking sequences. This vector is brought into the alkalophilic Bacillus strain. The vector is integrated into the chromosome via homologous recombination in the flanking region. Outrecombination and resolution leads to a Bacillus strain having the protease gene deleted. The vector used is preferably a plasmid. To make selection possible a selectable marker is introduced into the plasmid preferably an antibiotic resistance e.g. neomycin resistance. Preferably the vector can selectively be integrated into the chromosome this can be achieved by introducing an inducible origin of replication e.g. a temperature sensitive origin like the one from pE194. Said plasmid is introduced into a homologous host cell. Under high temperature condition, the plasmid is not able to replicate so that chromosomal integrants can be selected for. Integrants are selected at high temperatures in the presence of the relevant antibiotic (e.g. neomycin). Resolution of the plasmid from the host chromosome can leave the flanking regions in the chromosome while removing the coding region. Finally, the resulting mutant selected for has lost the selectable marker gene (is neomycin sensitive) and is protease negative. The final chromosomal configuration is determined by restriction analysis and Southern blotting. WO 88/06623 extensively describes the possible integration mechanisms. The protease gene, modified by site-directed mutagenesis is now introduced into the protease negative cell on a suitable expression vector. Care is taken that the plasmid carries short or preferably no sequences homologous to the chromosome to avoid plasmid instability.

Instead of expressing the mutated protease gene from a vector it is also possible to integrate the mutated protease gene into the chromosome of the protease negative strain. This can be achieved by leaving the flanking regions of the protease gene bordering the mutated protease gene in the plasmid. Introduction of such a plasmid may give rise to homologous recombination in the flanking regions thereby introducing the mutated protease gene in the chromosome of the protease negative strain. This is especially advantageous if the plasmid turns out be unstable. The present invention also shows that the amount of recombinant mutant or wild-type protease obtained is comparable or even higher if one copy of the gene is integrated in the genome then if there are several copies of the gene encoded by a plasmid.

In yet another embodiment the chromosomal high alkaline protease gene is replaced by the mutant gene by homologous recombination, without prior isolation of a protease negative strain.

The protease is expressed and can be isolated either from the cells or from the medium when the protein is secreted. After recovering the protease it can be purified and formulated into a detergent composition. Since, in the described production system the wild-type gene has been completely deleted, the mutant protease preparation is completely free from the wild-type protease.

Plasmid pM58, constructed as described in EP-A-0284126, contains the high alkaline protease gene. To obtain the inactivation plasmid the coding region of the protease gene is deleted by BalI/HpaI double digestion. The temperature-sensitive origin of replication from pE194neo-1 is subsequently introduced. The construct pEM58 Δ is introduced into Bacillus strain PBT110.

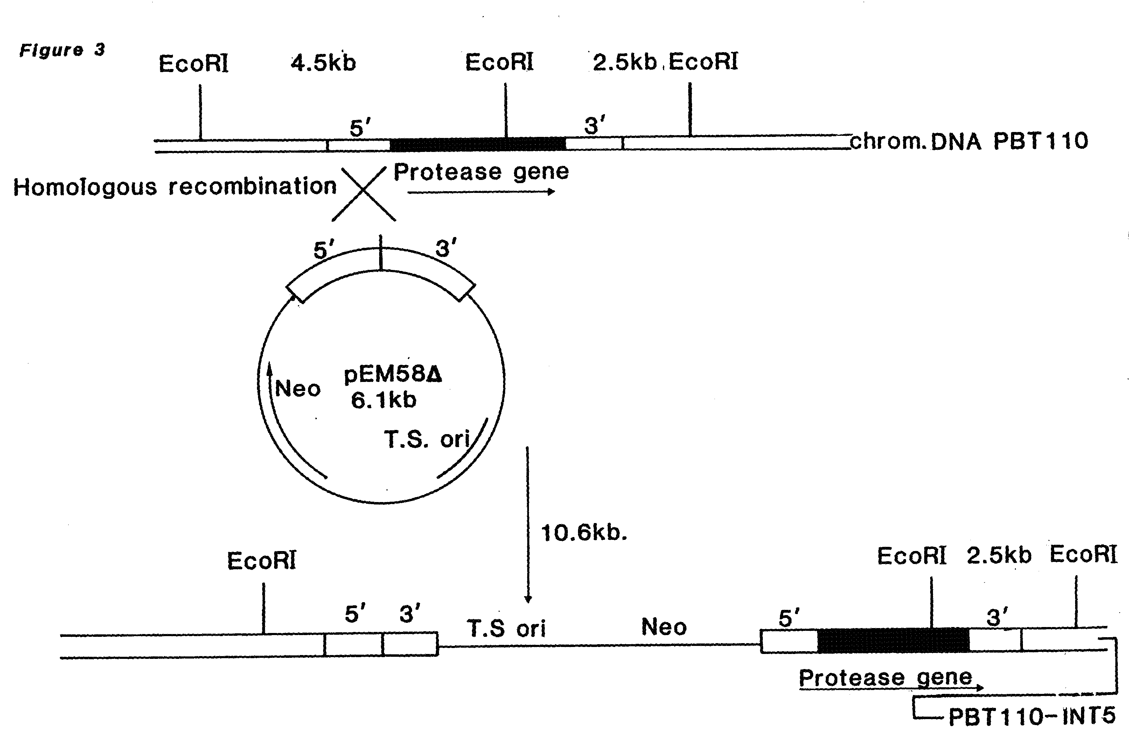

Integration by the so-called Campbell-type mechanism took place in the 5′ flanking region of the protease gene, resulting in a protease positive strain carrying the entire plasmid vector in its chromosome in the protease locus (as depicted in Figure 3).

Subsequent selection for protease negative derivatives of this strain revealed two strains PBT125 and PBT126, which had lost the protease gene and the neomycin marker. Further analysis showed that in this second step illegitimate recombination had occured.

Since the obtained Bacillus strains did not show a detectable protease activity they could be used for expression of altered protease genes. It will be clear that PBT125 and PBT126 are only examples. Since illegitemate recombination will give different results it will be essential to determine whether the complete gene has been deleted. To obtain more specific strains, homologous recombinants could have been made.

The examples given here mainly describe two specific embodiments of the invention. One is the production of plasmid encoded mutant protease from a protease negative strain. The other is the production of mutant protease whereby the mutated gene has been introduced in the genome by gene exchange. It will immediately be clear to someone skilled in the art that other embodiments are possible, for example; chromosomal integration in a protease negative strain, a mutated gene copy both on a plasmid and integrated into the chromosome, more than one copy of the mutated gene tandemly located or at different positions in the chromosome. The following examples are offered by way of illustration and not by way of limitation.

### EXPERIMENTAL SECTION

### EXAMPLE 1

### Construction of Inactivation Plasmid pEM58 Δ

Plasmid pM58 (see EP-A-0284126) was digested with restriction enzymes BalI and HpaI. The large fragment containing part of the flanking sequences of the protease gene was purified (Maniatis, Molecular cloning: A Laboratory Manual, Cold Spring Harbor 1982) and ligated. The ligation mix was transformed (Spizizen et al., J. Bacteriol. 81 (1961) 741-746) to Bacillus subtilis DB104 (Kawamura and Doi, J. Bacteriol. 160 (1984) 442-444) and selected for neomycin resistant and protease negative transformants on minimal plates containing 0.4% casein and 20 µg/ml neomycin.

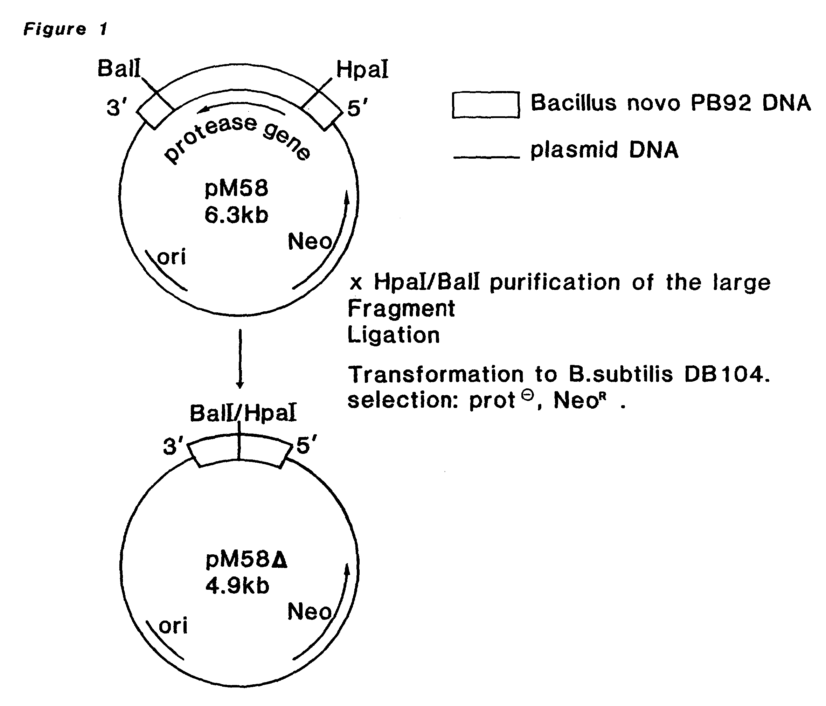

Plasmid pM58 Δ was isolated and characterized by restriction enzyme analysis. As shown in Figure 1 plasmid pM58 Δ contains the flanking sequences of the high alkaline protease gene, the gene coding for neomycin resistance and a Bacillus origin of replication.

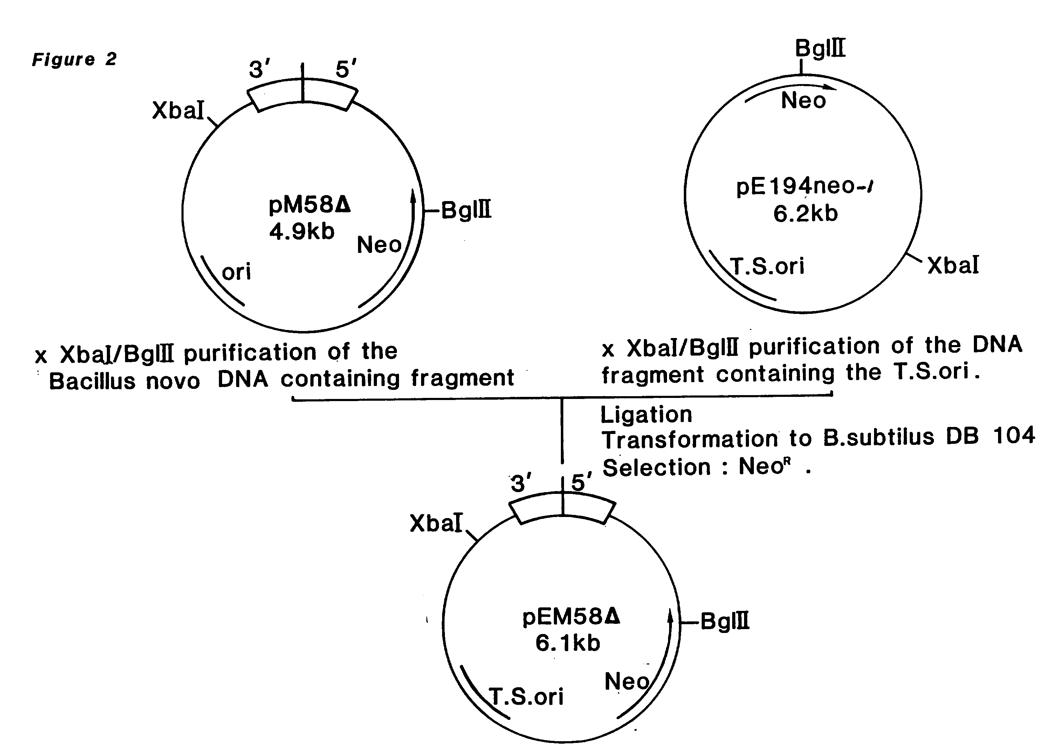

To construct an integration plasmid containing a temperature sensitive origin of replication, pM58 Δ was digested with restriction enzymes XbaI and BglII. The fragment containing the high alkaline protease flanking sequences was purified and ligated to the (purified) XbaI/BglII fragment of pE194neo-1 (EP-A-0284126) containing the temperature sensitive origin of replication, derived from pE194 (Jordanescu et al., Plasmid 1 (1978) 468-479) after said fragment was purified as formerly described. The ligation mix was transformed to B. subtilis DB104 and selected for neomycin resistance (see above). Plasmid pEM58 Δ was isolated and characterized (Figure 2). It contains the neomycin resistance gene, the temperature sensitive origin of replication from pE194 and the flanking sequences of the alkaline protease gene.

This plasmid pEM58 Δ was used to transform Bacillus strain PBT110.

### EXAMPLE 2

### Construction of a Protease Negative Alkalophilic Bacillus Strain

### A.Protoplast transformation of Bacillus PBT110 by plasmid pEM58Δ

Bacillus strain PBT110 is an asporogenous mutant of Bacillus novo species PB92 (U.S. Patent No. Re. 30,602) and was obtained by classical (UV) mutation procedures. This strain was transformed with plasmid pEM58 Δ similar to the method described in EP-A-0284126. Prior to integration experiments, it was checked by restriction enzyme analysis whether transformants contained the relevant plasmid.

### B.Integration of pEM58Δ in the Bacillus PBT110 chromosome

Integration of pEM58 Δ in the chromosome of Bacillus strain PBT110 was performed as described in EP-A-0284126. Selection for integrants occurred at a neomycin concentration of 1 µg/ml. The genetic organization of the integrant was determined by restriction enzyme analysis followed by Southern blotting and hybridization analysis and is shown in Figure 3. It appeared that integration of pEM58 Δ took place through a so-called Campbell-type mechanism by homologous recombination in the 5′ flanking region of the alkaline protease gene, resulting in Bacillus strain PBT110-INT5.

### C.Selection for protease negative strains

Bacillus strain PBT110-INT5 was inoculated in 100 ml tryptic soya broth (TSB) containing 1 µg/ml neomycin and incubated for 24 hours at 50°C.

After 24 hours 0.1 ml of the culture obtained was inoculated in a 500 ml shake flask containing 100 ml PBT minimal medium: K₂HPO₄, 17.42 g/l; glutamate, 13.36 g/l; sodium citrate.H₂O 2 g/l; FeSO₄.7H₂O, 0.05 g/l; ZnSO₄.7H₂O, 1 mg/l; MnSO₄.H₂O, 1 mg/l; CaCl₂.2H₂O, 10 mg/l; MgSO₄.7H₂O, 0.2 g/l; CuSO₄.5H₂O, 0.5 mg/l; H₃BO₃, 0.5 mg/l; Na₂MoO₄.2H₂O, 0.5 mg/l; CoCl₂.6H₂O, 0.5 mg/l; Biotine, 0.5 mg/l; saccharose, 20 g/l; (pH 8.0, sterilized for 20 min at 120°C). After sterilization 1 mg/l thiamine was added.

This culture was incubated for 4 days at 37°C. After 4 days, 1 ml of culture was diluted in 100 ml of the same medium and incubated for 4 days. After 4 days the culture was plated on PBT minimal medium plates containing additionally 0.4% casein and 15 g/l agar. Colonies which lacked a detectable protease halo were considered protease minus and were checked for the absence of the gene encoding the PBT110 alkaline protease.

### D.Characterization of protease negative strains

Strains which did not show a detectable halo on the casein plates described in the previous section were primarily tested for the presence of a neomycin sensitive and asporogenous phenotype. Two strains, Bacillus PBT125 and PBT126, which contained the protease negative and asporogenous phenotype were tested for protease production in 500 ml shake flasks containing 100 ml protease production medium, as described in U.S. Patent No. Re. 30,602. Neither strain produced detectable amounts of protease.

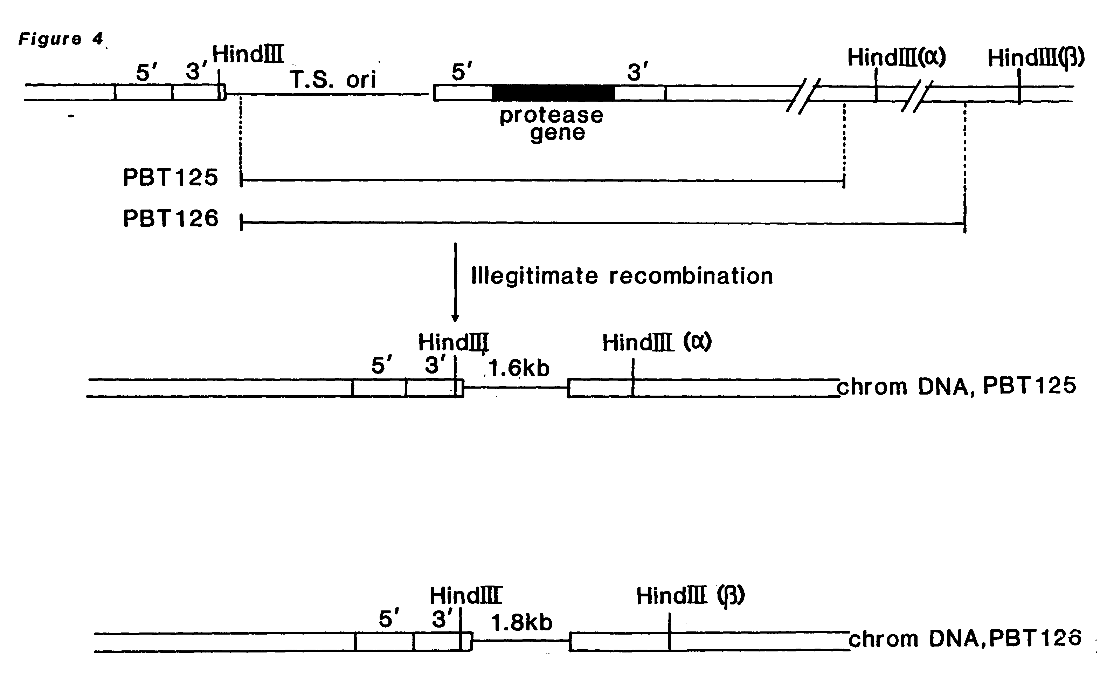

The genetic organization of the two strains was determined by restriction enzyme analysis and chromosomal blotting and is shown in Figure 4. No homologous but illegitimate recombination had occurred, resulting in two strains with completely deleted protease and neomycin resistance genes. However, a small plasmid fragment appeared to be left in the chromosome after deletion of the protease gene. The sequence of this fragment was determined as follows. The chromosomal HindIII fragments of strains PBT125 and PBT126 containing the plasmid/chromosome junctions were ligated in phage vector M13 mp18 (Messing et al., Nucl. Acids Res. 9 (1981) 303-321) and transfected to E. coli JM101 according to the procedure described by Cohen et al., Proc. Natl. Acad. Sci. USA 69 (1972) 2110-2114. After phage propagation in E. coli JM101, ssDNA was isolated (Heidecker et al., Gene 10 (1980) 69-73).

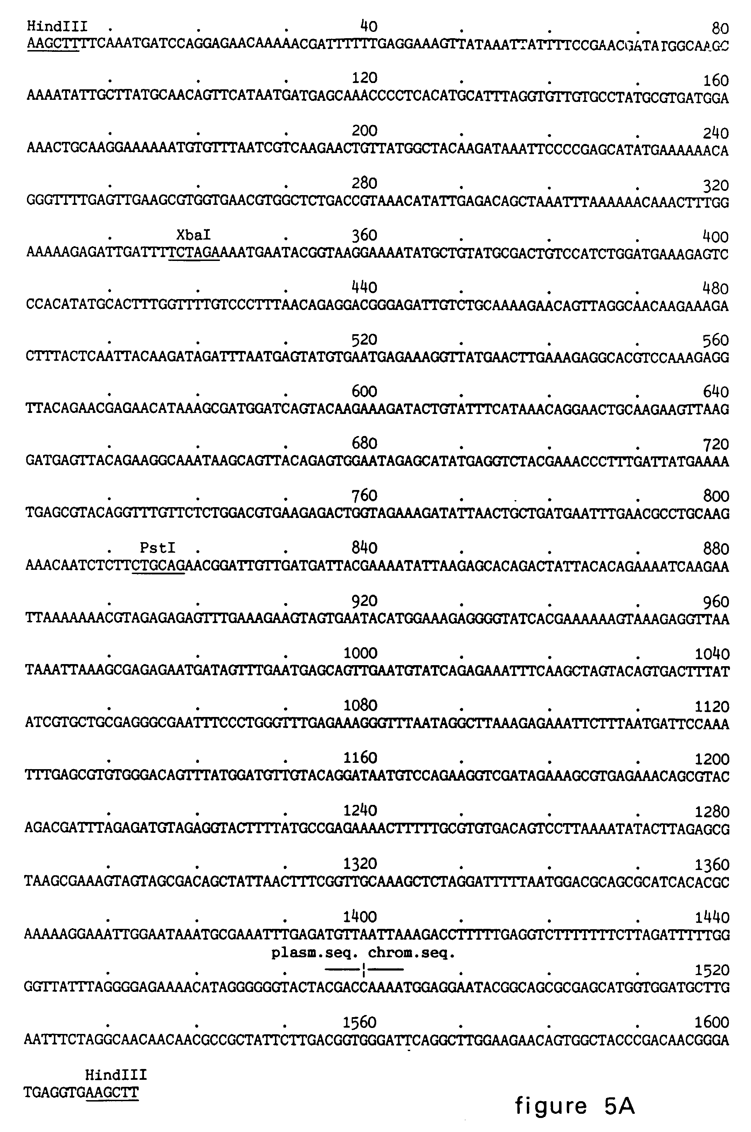

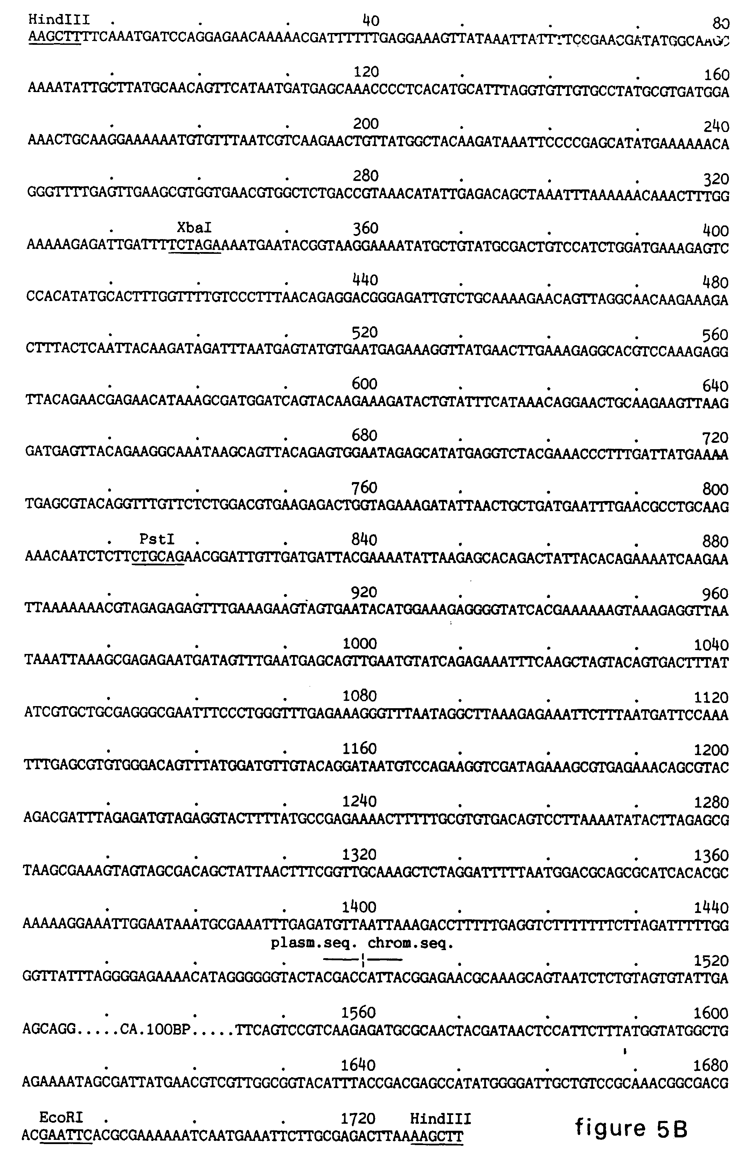

The inserts were sequenced using the method described by Sanger et al., Proc. Natl. Acad. Sci. USA 74 (1977) 6463. The results are shown in Figures 5A and 5B, respectively.

### EXAMPLE 3

### Construction of Protease Production and Mutation Vectors

### A. Subcloning of the PB92 protease gene in phage M13 mp18

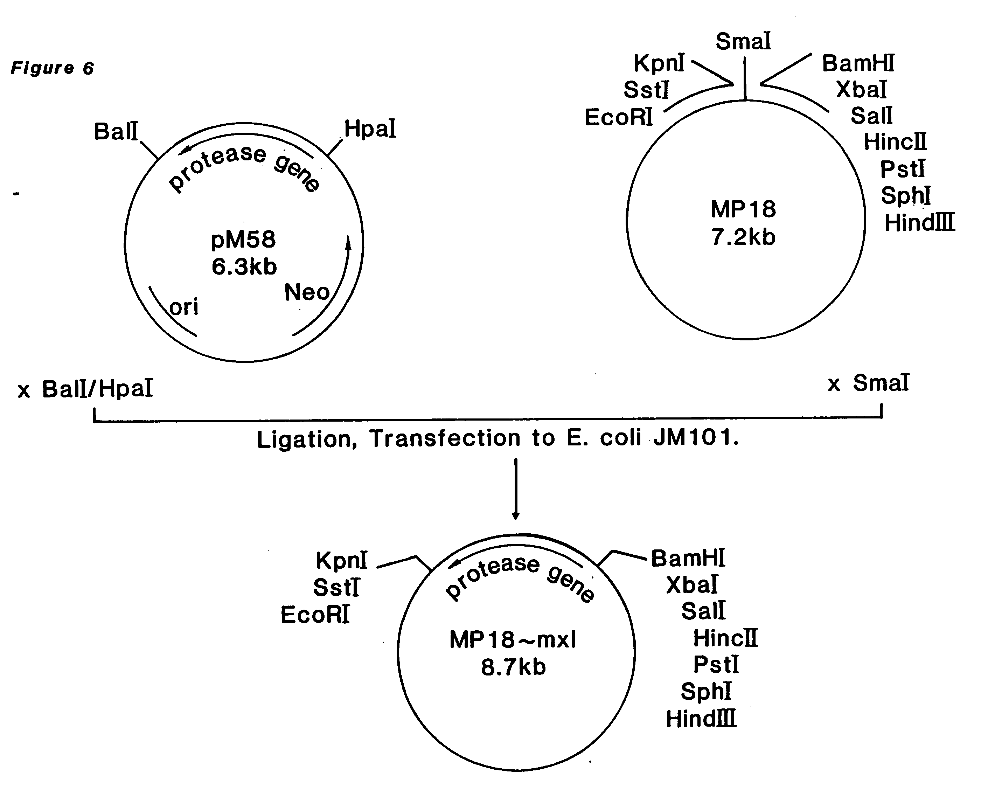

Plasmid pM58 was digested with HpaI and BalI. After purification of the DNA fragment containing the protease gene (Maniatis, 1982) this fragment was ligated into phage M13 mp18 which was digested with SmaI. The ligation mixture was transfected to E. coli JM101 (Cohen et al., supra). After phage propagation in E. coli JM101, dSDNA was isolated according to the method described by Birnboim and Doly (Nucl. Acids Res. 7 (1979) 1513-1523). The insert and its orientation were checked by restriction enzyme analysis. Vector mp18_{∼}MXL was used for further subcloning experiments and is shown in Figure 6.

### B. Subcloning of the PB92 protease gene in plasmid pBHA1

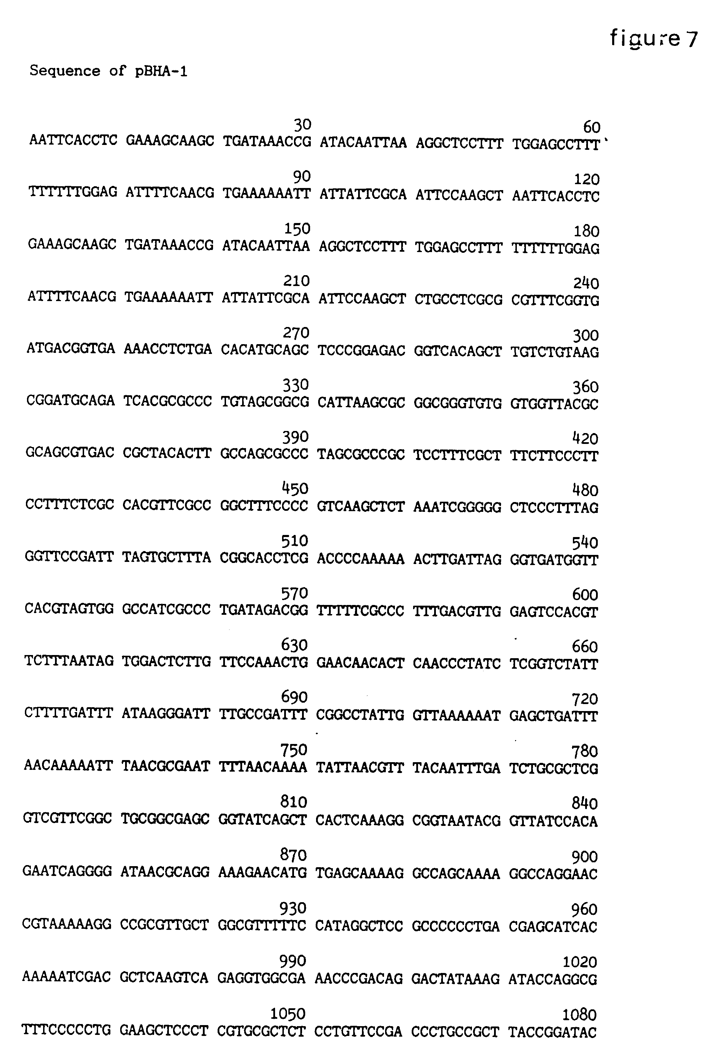

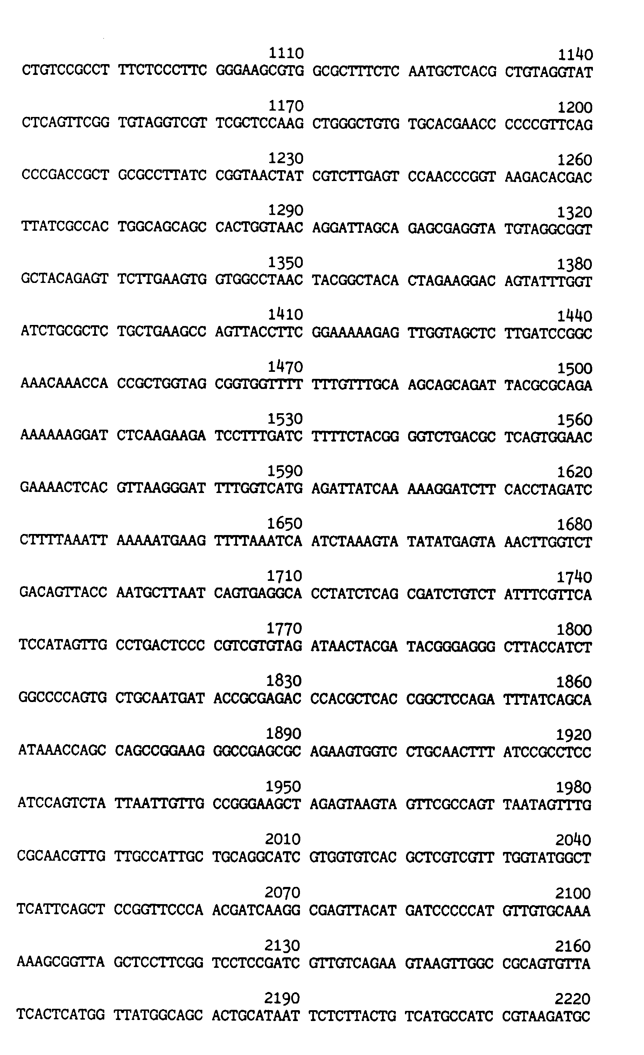

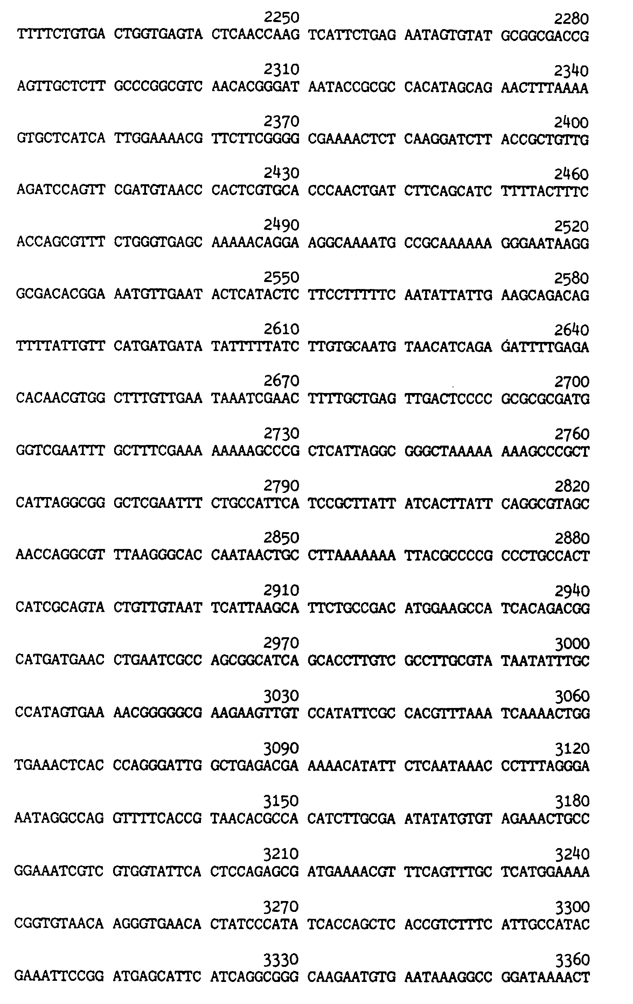

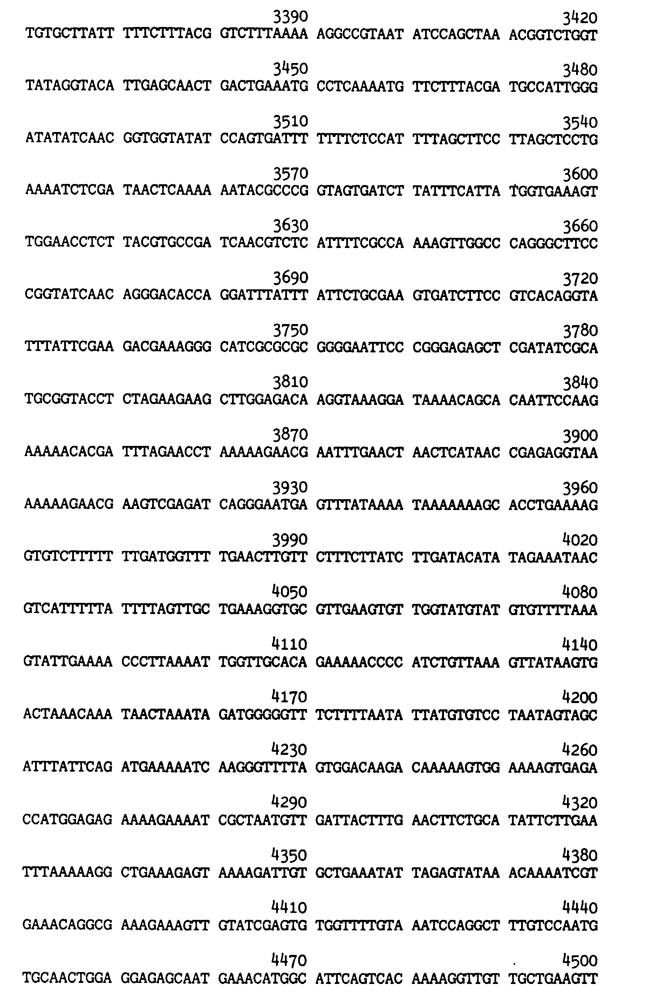

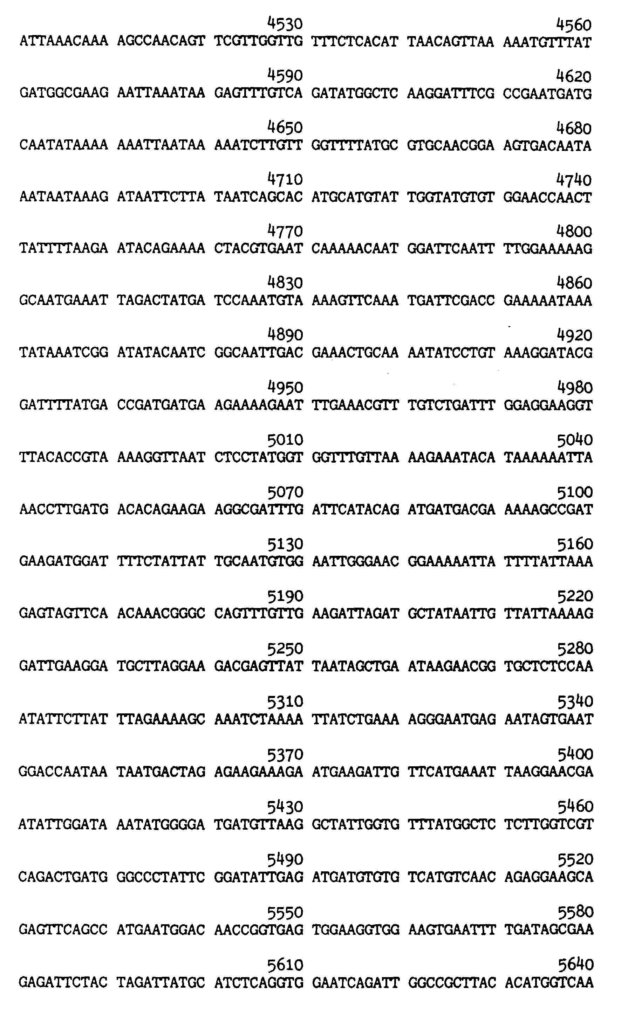

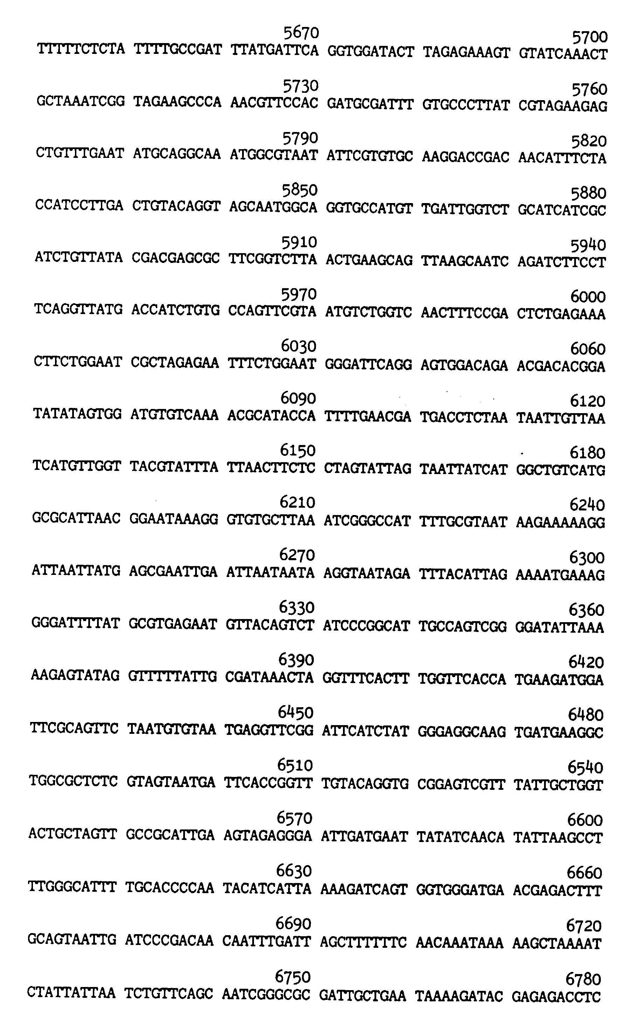

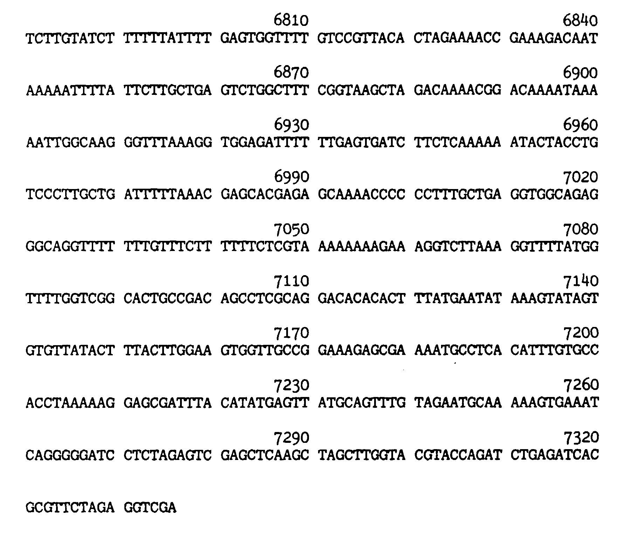

Figure 7 shows the nucleotide sequence of plasmid pBHA1. This plasmid is derived from the twin vector system pMa/c5-8, described by Stanssens et al. (Nucl. Acids Res. 17 (1989) 4441-4454) in which an additional promoter was inserted. Plasmid BHA1 consists of the following fragments:
- pos 11-105:: bacteriophage FD, terminator;
- pos 121-215:: bacteriophage FD, terminator;
- pos 221-307:: a part of plasmid pBR322 (viz. positions 2069-2153);
- pos 313-768:: bacteriophage F1, origin of replication (viz. pos 5482-5943);
- pos 772-2571:: part of plasmid pBR322, viz. the origin of replication and the β-lactamase gene;
- pos 2572-2685:: transposon Tn903, complete genome;
- pos 2719-2772:: tryptophan terminator (double);
- pos 2773-3729:: transposon Tn9, the chloramphenicol-acetyltransferase (=cat) gene. The nucleotides at pos 3005(A), 3038(C), 3302(A) and 3409(A) differ from the wild-type cat coding sequence. The mutations were introduced to eliminate the NcoI, BalI, EcoRI and PvuII sites;
- pos 3730-3804:: multiple cloning site;
- pos 3807-7264:: part of plasmid pUB110 (viz. the replication function and kanamycin resistance gene, EcoRI-PvuII fragment) (McKenzie et al., Plasmid 15 (1986) 93-103 and Plasmid 17 (1987) 83-85);
- pos 7267-7331:: multi cloning site.

The fragments were combined using known cloning techniques, e.g., filling in sticky ends with Klenow, adapter cloning, etc. All data were derived from Genbank^{R} National Nucleic Acid Sequence Data Bank NIH, U.S.A. Plasmid pMc5-8 was deposited under DSM 4566.

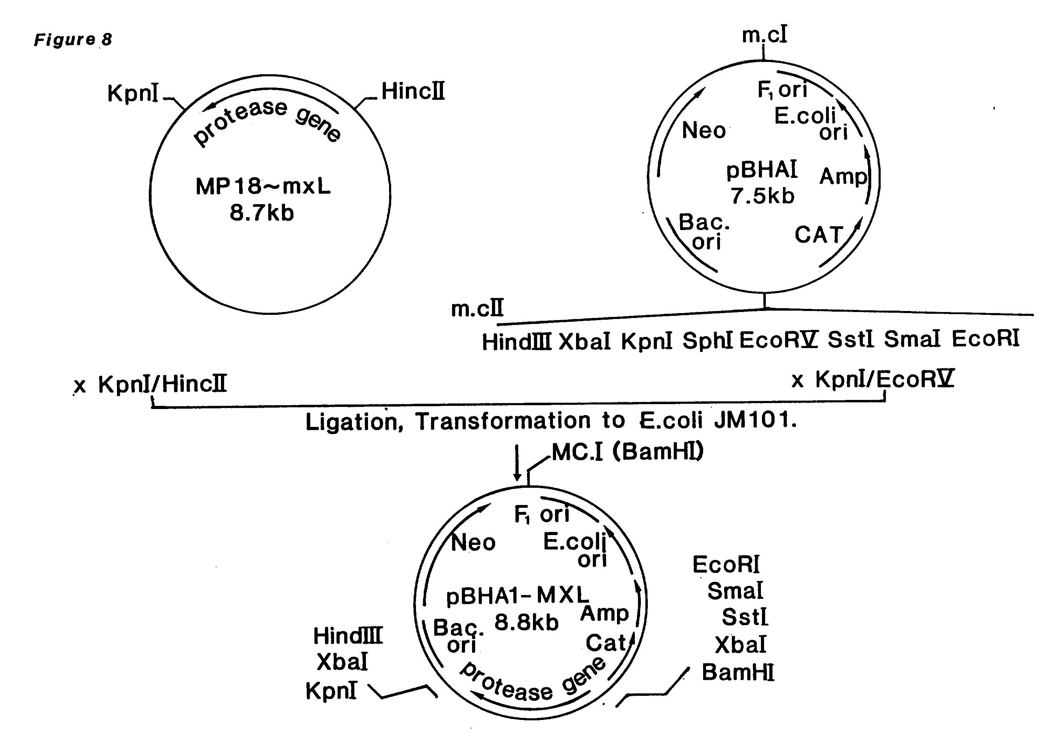

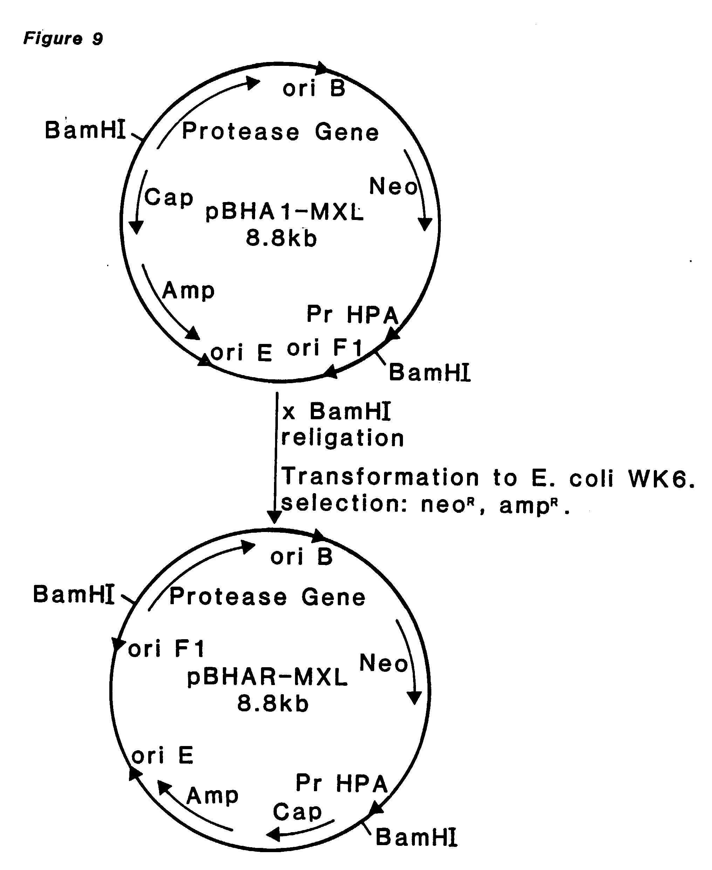

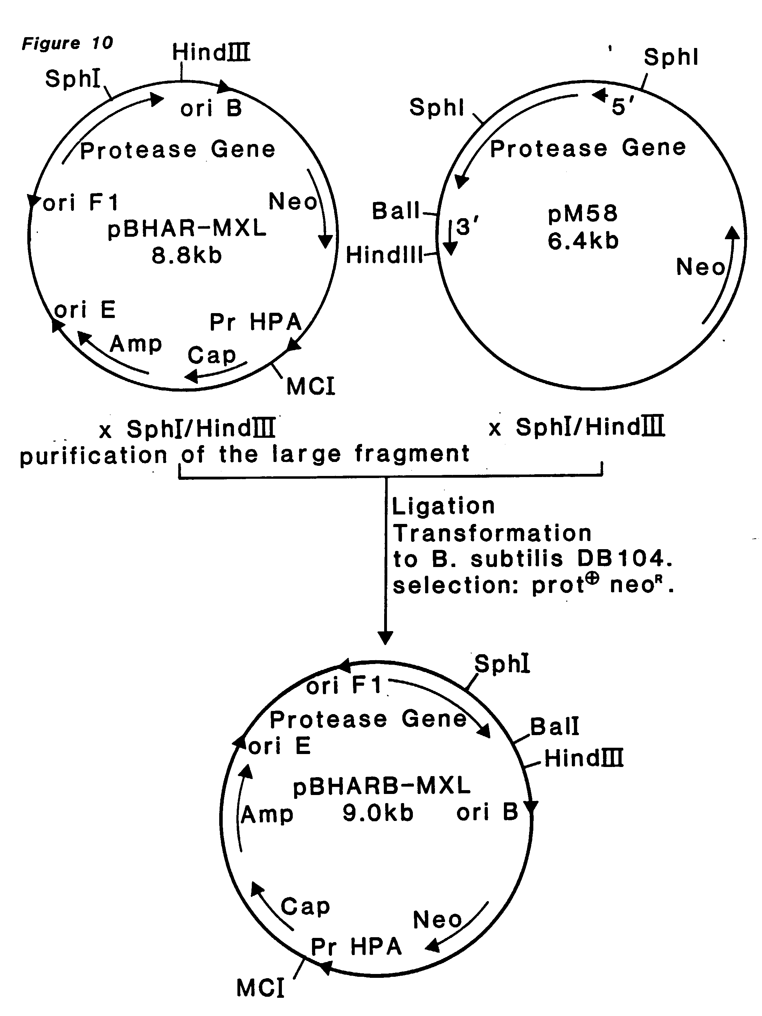

Prior to mutation procedures the twin vector system pBHA/C-1 was modified to obtain the twin vector system pBHARB-MXL/pBHCRB-MXL, as follows:
1. Vector mp18_{∼}MXL was digested with KpnI and HincII. The DNA fragment containing the protease gene was purified and ligated into pBHA1 which was digested with EcoRV and KpnI. The ligation mixture was transformed to E. coli JM101 (Maniatis, 1982) and plated on LC⁺ plates containing 10 g/l trypton, 5 g/l Yeast extract (Difco), 8 g/l NaCl, 25 mg/l thymine, 1 g/l MgSO₄.7H₂O, 100 µg/ml ampicillin and 20 µg/ml neomycin pH 7.0. Plasmid DNA of transformants was isolated (Birnboim, supra) and characterized by restriction enzyme analysis. In this way pBHA1-MXL was isolated (see Figure 8).
2. In order to sequence mutations introduced in the protease gene with an available set of oligonucleotides, it was necessary to reverse the orientation of the F1 origin compared to the protease gene. This was performed in the following way: plasmid pBHA1-MXL was digested with BamHI and religated. The ligation mix was transformed to E. coli WK6 (Maniatis, supra) and selected for neomycin or ampicillin resistance on LC⁺ plates containing 10 g/l trypton, 5 g/l Yeast Extract (Difco), 8 g/l NaCl, 25 mg/l thymine, 1 g/l MgSO₄.7H₂O, 100 µg/ml ampicillin and 20 µg/ml neomycin pH 7.0. Plasmid DNA of transformants was isolated (Birnboim, supra) and characterized by restriction enzyme analysis. In this way pBHAR-MXL was isolated, which differs from pBHA1-MXL in that the orientation of the E. coli sequence is reversed, (see Figure 9).
3. For the introduction of additional flanking sequences in plasmid pBHAR-MXL, the following digestions were made. Plasmid pM58 was digested with SphI and HindIII. Plasmid pBHAR-MXL (Figure 10) was digested with SphI and HindIII. The larger fragment was purified. Both digests were ligated and transformed to B. subtilis DB104 and selected for neomycin resistance and protease activity on minimal plates containing 10 µg/ml neomycin and 0.4 % casein. Transformants were characterized by restriction enzyme analysis. One of these, pBHARB-MXL (Figure 10), was used for further experiments.

### C. Mutagenesis of the PB92 gene in plasmid pBHARB-MXL

Mutagenesis was carried out with the twin vector system pBHARB-MXL/pBHCRB-MXL (Stanssens, supra).

A method based on the gapped-duplex approach (Kramer et al., Nucl. Acids Res. 12 (1984) 9441) and a phasmid (phage/plasmid hybrid) was used. Essentially the method rests on a gapped-duplex DNA intermediate consisting of a gapped strand (- strand) containing a wild-type antibiotic resistance marker and a template strand (+ strand) carrying an amber mutation in the gene conferring resistance to the antibiotic. After annealing, the mutagenic oligonucleotide becomes incorporated in the gapped strand during in vitro gap-filling and sealing reaction. The resultant molecules are used to transform a mismatch repair deficient (Mut S) host in which the linkage between the intended mutation and the antibiotic resistance marker is preserved. The mixed phasmid population, isolated from this strain, is then allowed to segregate in a suppressor negative host strain. Transformants are plated on antibiotic containing medium, thus imposing a selection for progeny derived from the gapped strand.

In the pMa type vector nucleotide 3409 is changed from G to A, while in the pMc type vector nucleotide 2238 is changed from G to C, creating amber stopcodons in the chloramphenicol-acetyltransferase gene and β-lactamase gene, respectively, rendering said genes inactive.

To perform mutagenesis the target DNA fragment is cloned into the multiple cloning site of pMa5-8 or a derivative thereof. A gapped duplex between pMa5-8 containing the target DNA and pMc5-8 is then constructed.

The single strand gap, consisting of the target DNA, may be subjected to mutagenesis with a mutagenic oligonucleotide, with long synthetic oligonucleotides having a low level of misincorporated nucleotides, using chemical or enzymatic misincorporation of nucleotides. For a detailed description, see Ausubel et al., 1987, Current Protocols in Molecular Biology, John Wiley & Sons Inc. New York; or B. Perbal, 1988, A practical Guide to Molecular Cloning, 2nd ed. John Wiley & Sons Inc; New York.

### D. Construction of Vectors for Industrial Protease Production

After the introduction of the desired mutation(s) in plasmid pBHARB-MXL, the unwanted E. coli sequences from the plasmid were deleted as follows: plasmid pBHARB-MXL containing the relevant mutation was digested with BamHI and religated under diluted conditions. The ligation mixture was transformed to B. subtilis DB104 and selected for neomycin resistance and protease activity on minimal plates containing 20 µg/ml neomycin and 0.4 % casein. DNA of transformants was isolated and characterized by restriction enzyme analysis. In this way vectors were isolated lacking E. coli DNA, which are suitable for commercial production of proteins.

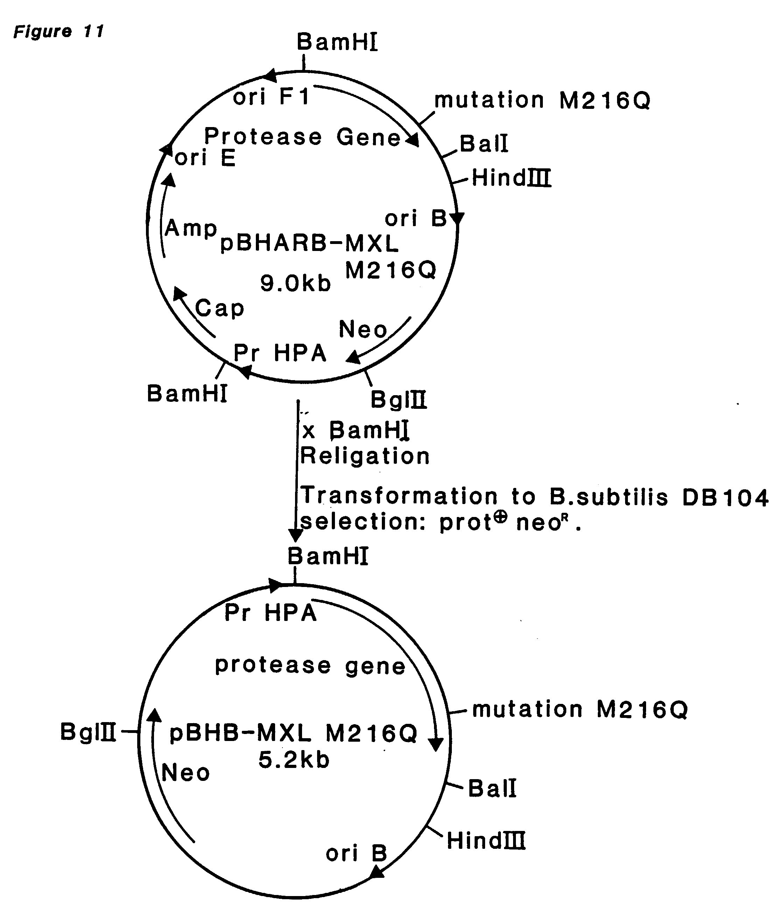

The foregoing procedure is illustrated in Figure 11 where mutation M216Q is taken as an example, resulting in plasmid pBHB-MXL M216Q. M216Q, and also M216S, S160D and N212D referred to hereinafter, are mutant proteases of Bacillus PB92, described in EP-A-0328229.

### EXAMPLE 4

### Protoplast Transformation of Bacillus PBT125 by Plasmid pBHB-MXL Derivatives

Bacillus strain PBT125 was transformed with plasmid pBHB-MXL or plasmids derived thereof containing mutant protease genes, following the transformation procedure described in EP-A-0284126. Prior to production studies, transformants were checked by restriction enzyme analysis to contain the relevant plasmid.

### EXAMPLE 5

### Construction of Protease Integration Vectors

### A. Integration vector pEN₃Q

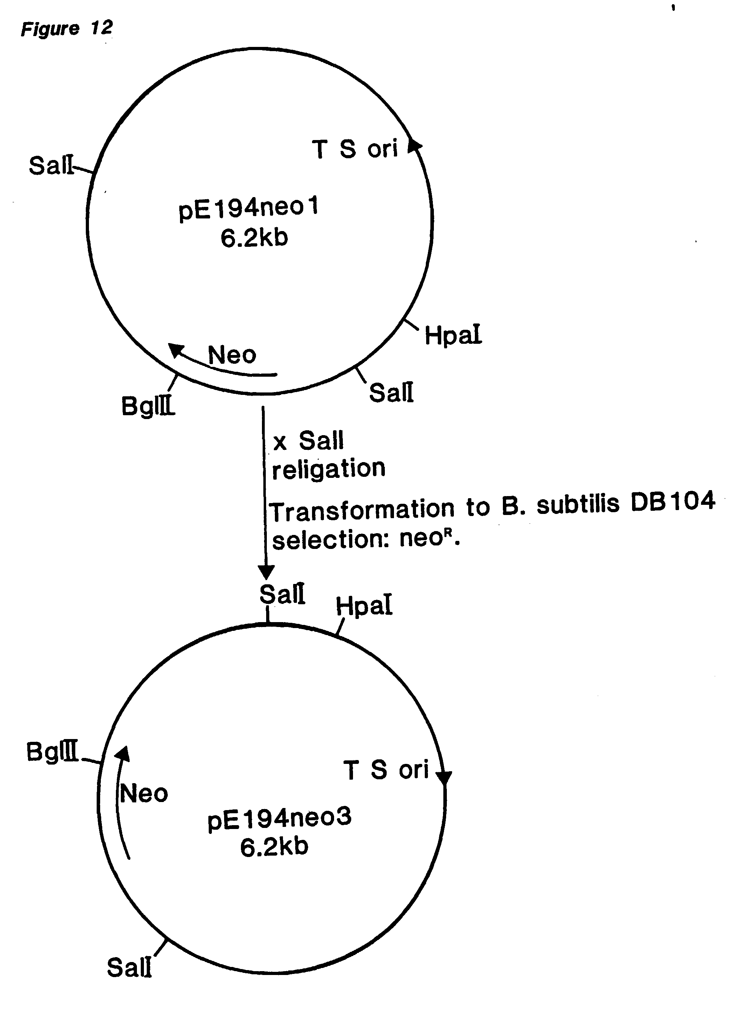

Plasmid pE194neo-1 (EP-A-0284126) was digested with SalI and religated. The ligation mix was transformed to B. subtilis DB104 and selected for neomycin resistance on minimal plates containing 20 µg/ml neomycin. Plasmid pE194 neo-3 was isolated and checked on the presence of the neomycin resistance gene in a reversed orientation (see Figure 12). Plasmid pE194neo-3 was digested with HpaI and BglII. The fragment containing the temperature sensitive origin of replication was purified (Maniatis, supra).

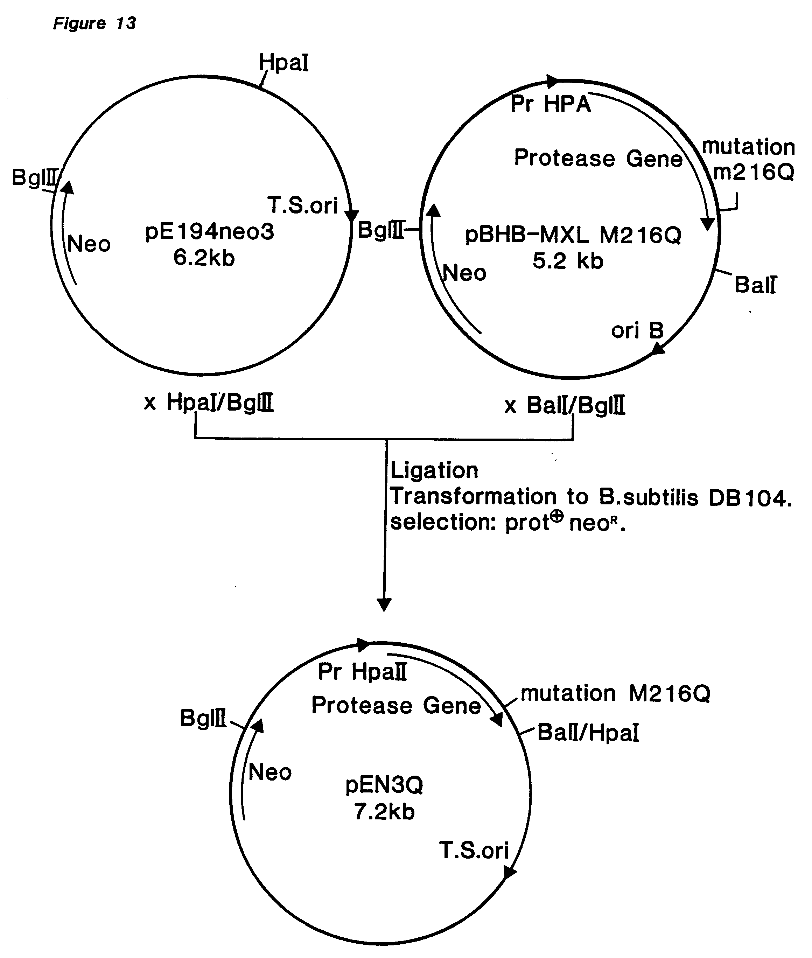

Plasmid pBHB-MXL M216Q (see Example 3 and Figure 11) was digested with BglII and BalI. The fragment containing the protease gene was purified. Both fragments were ligated. The ligation mix was transformed to B. subtilis DB104 and selected for protease activity and neomycin resistance as described before. Transformants were characterized by restriction enzyme digestions. One of these, containing plasmid pEN₃Q, was selected (Figure 13). This plasmid contained the neomycin resistance gene, the t.s. origin of replication from plasmid pE194neo-3 and the M216Q mutation in the PB92 protease gene.

### B. Integration vector pEN₃S.

In the same way as described for integration vector pEN₃Q, pEN₃S was constructed using pBHARB-MXL M216S as starting vector.

### EXAMPLE 6

### Construction of Bacillus Strains PEP111 and PEP112

### A. Protoplast transformation of Bacillus PBT110 by Plasmid pEN₃Q

Bacillus strain PBT110 was transformed with plasmid pEN₃Q as described in EP-A-0284126. Prior to integration experiments, it was checked by restriction enzyme analysis whether the transformants contained the relevant plasmid.

### B. Integration of pEN₃Q in Bacillus PBT110 chromosome

Integration experiments with plasmid pEN₃Q in Bacillus strain PBT110 chromosome were performed as described in EP-A-0284126. Selection for integrants occurred at a neomycin concentration of 1µg/ml at non-permissive temperatures (50°C) for plasmid replication. To check the integration of PEN₃Q in the chromosome, chromosomal DNA of potential integrants was isolated, digested with HindIII or ClaI, run on a 0.8% DNA agarose gel and blotted to nitrocellulose (Southern, J. Mol. Biol. 98 (1975) 503-517) and hybridized with ³²P labeled nick-translated pEN₃Q DNA (Maniatis, 1982).

It was shown that integration of pEN₃Q had occurred by homologous recombination resulting in a strain (PBT11OM/Q) with two protease genes (one wild type and one mutant M216Q) tandemly located on the chromosome. Thus, integration of pEN₃Q occurred by a so-called Campbell-type mechanism as depicted in Figure 14 (see also EP-A-0284126).

### C. Selection of neomycin sensitive recombinants

Neomycin sensitive recombinants were selected, which contain either the wild-type or the mutant protease gene in the chromosome (hereinafter called "outrecombinants"). The selection procedure is similar to the procedure described before in Example 2C.

Strain PBT110 M/Q was used as starting strain. After strain PBT110 M/Q was incubated in PBT minimal medium, the culture was plated on PBT minimal medium plates containing 0.4% casein and 15 g/l agar. These plates were incubated for 48 h at 37°C and replica plated to Heart infusion (HI) plates containing 1 µg/ml neomycin and no neomycin, respectively. Neomycin sensitive colonies were considered outrecombinants.

### D. Characterization of neomycin sensitive recombinants.

Chromosomal DNA of potential outrecombinants was isolated, digested with ClaI or HindIII, run on a 0.5% agarose gel, blotted to nitrocellulose (Southern, 1975) and hybridized with ³²P labeled nick translated pEN₃Q (Maniatis, 1982). Since mutation M216Q results in the removal of a ClaI site, the strains containing a wild-type protease or a mutant protease gene, and the intermediate strain PBT110 M/Q can be easily distinguished, see Fig. 14.

An outrecombinant containing the M216Q mutant protease gene of PB92 was isolated. After it was shown that a gene replacement of PB92 wild-type [protease to PB92 mutant protease had occurred, the product of the strain was characterized by its biochemical parameters (k_{cat}, Kₘ), oxidation resistance, specific activity and wash performance, as described EP-A-0328229. All results were equal to a control protease M216Q, indicating that the product of the transformed strain was indeed the PB₉₂ protease containing the mutation M216Q. This transformed strain is called Bacillus PEP111.

Similar experiments were performed using vector pEN₃S to construct Bacillus strain PEP 112, which contains the PB92 protease with mutation M216S.

### EXAMPLE 7

### Construction of Bacillus Strains PEP211 and PEP212

Bacillus strain PEP111 was transformed with plasmid pEN₃Q following the procedure described in EP-A-0284126. Prior to the integration procedure, it was checked by restriction enzyme analysis whether the transformants contained the relevant plasmid. The integration procedure and the selection for integrants (at a neomycin concentration of 20 µg/ml at non-permissive temperatures (50°C) for plasmid replication) was performed as described in EP-A-0284126.

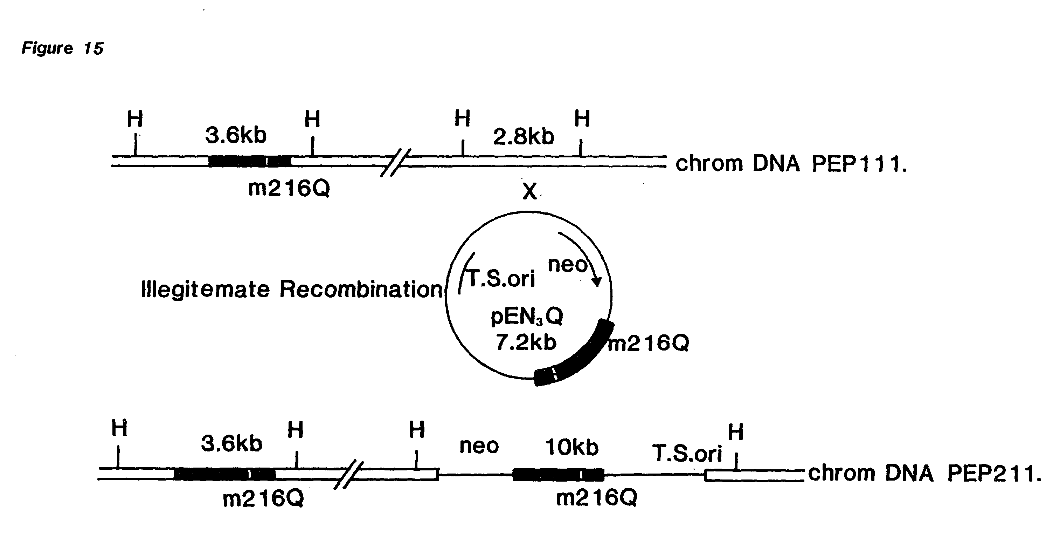

To check the integration of plasmid pEN₃Q in the chromosome, chromosomal DNA of potential integrants was isolated and digested with HindIII, run on 0.8% agarose gels, blotted to nitrocellulose (Southern et al. 1979) and hybridized with 3²p labeled nick translated pEN₃Q. It was shown that a strain containing two mutant (M216Q) PB92 protease genes, separately located on the chromosome was isolated. This strain is called PEP211. Its chromosomal organization is shown in Figure 15.

To obtain an analogous strain containing two protease genes with mutation M216S, strain PEP112 containing a mutant gene (M216S) after gene replacement of the wild-type gene, was transformed with plasmid pEN₃S and procedures were followed as described above. These experiments resulted in strain PEP212 containing two separately located PB92 mutant M216S protease genes on the chromosome.

### EXAMPLE 8

### Production of Mutant Proteases

Production of protease mutants was performed with transformed Bacillus strains in which the mutated protease encoding gene was either localized on a plasmid or on the chromosome.

For plasmid encoded production 2 types of strains were used, the alkalophilic strain Bacillus PBT125, as herein described, and B. subtilis strain DS12367, an asporogenous strain derived from B. subtilis DB104 (Kawamura et al., J. Bacteriol. (1984) 160, 442-444). These strains were transformed with pBHB-MXL derived plasmids. Bacillus PBT125 and B. subtilis DS12367, which do not possess protease encoding plasmids, were used as control strains.

For production with strains containing chromosomally integrated protease mutant genes the Bacillus PEP strains, as herein described, were used. Bacillus PBT strains 108 and 110, containing the wild-type protease genes were used as control strains. PBT108 is a strain containing two wild-type genes separately located on the chromosome, see EP-A-0284126. For PBT110, see Example 2A.

To check the activity of the original promoter of the PB92 protease gene in B. subtilis, a construct was made, pBHB-MXLR, in which the orientation of the protease gene to the HPaII promoter (Zyprian et al., DNA 5 (1986) 219-225) was reversed, so that the expression of the protease gene is only directed by its original promoter.

The productions were performed in 500 ml shake flasks containing 100 ml protease production medium.

When alkalophilic Bacillus strains were used cultures were incubated, after inoculation, for 40 hours at 37°C in a production medium as described in U.S. Patent No. Re. 30,602. In case of plasmid containing strains, neomycin (20 µg/ml) was added.

When B. subtilis DS12367 strains were used, a comparable medium containing 12.5 g/l Yeast Extract (Difco), 0.97 g/l CaCl₂.6H₂O, 2.25 g/l MgCl₂.6H₂O, 20 mg/l MnSO₄.4H₂O, 1 mg/l CoCl₂.6H₂O, 0.5 g/l citrate, 0.5 ml antifoam 5693, 6% w/w maltose, 0.2 M phosphate buffer pH 6.8 was applied. In case of plasmid containing strains, neomycin (20 µg/ml) was added.

Cultures containing B. subtilis strains were incubated for 65 hours at 37°C. In all cases the shake-flasks were inoculated with 0.1 ml of a Tryptic Soya Broth culture containing 20 µg/ml neomycin containing the relevant strain which had been incubated for 24 hours at 37°C.

Protease activity was assayed using dimethylcasein as substrate as described by Lin et al., J. Biol. Chem. 244 (1969) 789-793. The specific activities of the protease mutants (EP-A-0328229) were used to determine the production on mg basis.

The results of the fermentation experiments are summarized in the following Table 1.

**Table 1**

| Strain | Relative protease production | Mutation |
|---|---|---|
| PBT110 | 100 % | WT |
| PBT125 | 0 % | - |
| PBT108 | 120 % | WT |
| PEP111 | 100 % | M216Q |
| PEP112 | 100 % | M216S |
| PBT125 pBHB-MXL | 95-100 % | WT |
| PBT125 pBHB-MXL M216Q | 95-100 % | M216Q |
| PBT125 pBHB-MXL M216S | 95-100 % | M216S |
| PBT125 pBHB-MXL S160D | 95-100 % | S160D |
| PBT125 pBHB-MXL N212D | 95-100 % | N212D |
| PEP211 | 120 % | M216Q |
| PEP212 | 120 % | M216S |
| DS12367 | 0-1 % | - |
| DS12367 pBHB-MXLR | 4 % | WT |
| DS12367 pBHB-MXL | 40 % | WT |
| DS12367 pBHB-MXL M216Q | 40 % | M216Q |
| DS12367 pBHB-MXL M216S | 40 % | M216S |
| DS12367 pBHB-MXL S160D | 40 % | S160D |
| DS12367 pBHB-MXL N212D | 40 % | N212D |

All publications (including patent applications) mentioned in this specification are indicative to the level of skill of those skilled in the art to which this invention pertains. All publications are herein incorporated by reference to the same extent as if each individual publication was specifically and individually indicated to be incorporated by reference.

Although the foregoing invention has been described in some detail by way of illustration and example for purposes of clarity of understanding, it will be apparent to one of ordinary skill in the art that many changes and modifications can be made thereto without departing from the spirit or scope of the appended claims.

## Claims

1. A method for the production of proteases differing in at least one amino acid from the wild-type protease, said method comprising the use of a homologous alkalophilic Bacillus strain as expression host said strain being unable to produce the wild-type protease, whereby from said Bacillus strain the wild-type high alkaline protease gene is deleted by replacing said wild-type high alkaline protease gene by at least one copy of the mutant high alkaline protease gene or whereby from said Bacillus strain the wild-type high alkaline protease gene is deleted in such a way that the sequences left in the chromosome are too short for homologous recombination with plasmid encoded protease gene and wherein at least one copy of a mutant high alkaline protease gene is inserted into the genome or is plasmid encoded.

2. A method according to Claim 1, employing as expression host a protease negative alkalophilic Bacillus strain.

3. A method according to any one of Claims 1 or 2, employing a protease negative derivative of Bacillus novo species PB92.

4. A method according to any one of Claims 1 to 3, employing an asporogenous alkalophilic Bacillus strain.

5. A method according to any one of Claims 1 to 4, employing an asporogenous Bacillus from which the wild-type protease gene has been deleted by homologous or illegitimate recombination.

6. A method according to Claim 1, wherein the wild-type high alkaline protease has essentially the amino acid sequence of Bacillus novo species PB92 protease.

7. A method of obtaining an alkalophilic Bacillus strain capable of production of proteases differing in at least one amino acid form the wild-type protease, whereby from a Bacillus strain the wild-type high alkaline protease gene is deleted by replacing said wild-type high alkaline protease gene by at least one copy of the mutant high alkaline protease gene or whereby from said Bacillus strain the wild-type high alkaline protease gene is deleted in such a way that the sequences left in the chromosome are too short for homologous recombination with plasmid encoded protease gene and wherein at least one copy of a mutant high alkaline protease gene is inserted into the genome or is plasmid encoded.

8. A method according to Claim 7, wherein said alkalophilic Bacillus strain is Bacillus novo species PB92 or a derivative thereof.

## Patentansprüche

1. Verfahren zur Herstellung von Proteasen, die sich in mindestens einer Aminosäure von der Wildtyp-Protease unterscheiden, umfassend die Verwendung eines homologen, alkalophilen, zur Bildung der Wildtyp-Protease nicht befähigten Bacillus-Stamms als Patentansprüche Expressionswirt, wobei in besagtem Bacillus-Stamm das Wildtyp-Gen für die hochalkalische Protease durch Austausch dieses Wildtyp-Gens für die hochalkalische Protease durch mindestens eine Kopie der Genmutanten für hochalkalische Protease deletiert worden ist, oder wobei in besagtem Bacillus-Stamm das Wildtyp-Gen für die hochalkalische Protease so deletiert worden ist, dass die im Chromosom verbleibenden Sequenzen für eine homologe Rekombination mit einem Plasmid-kodierten Protease-Gen zu kurz sind und worin mindestens eine Kopie einer Genmutanten für hochalkalische Protease in das Genom eingefügt oder Plasmid-kodiert ist.

2. Verfahren gemäss Anspruch 1, wobei als Expressionswirt ein Protease-negativer alkalophiler Bacillus-Stamm verwendet wird.

3. Verfahren gemäss einem der Ansprüche 1 oder 2, wobei ein Protease-negativer Abkömmling der Novo-Art PB92 von Bacillus eingesetzt wird.

4. Verfahren gemäss einem der Ansprüche 1 bis 3, wobei ein asporogener alkalophiler Bacillus-Stamm eingesetzt wird.

5. Verfahren gemäss einem der Ansprüche 1 bis 4, wobei ein asporogener Bacillus eingesetzt wird, in dem das Wildtyp-Gen für die Protease durch homologe oder illegitime Rekombination deletiert worden ist.

6. Verfahren gemäss Anspruch 1, in dem die hochalkalische Wildtyp-Protease im wesentlichen die Aminosäuresequenz der Protease der Novo-Art PB92 von Bacillus aufweist.

7. Verfahren zur Herstellung eines alkalophilen Bacillus--Stamms, der zur Bildung von Proteasen befähigt ist, die sich in mindestens einer Aminosäure von der Wildtyp-Protease unterscheiden, wobei von einem Bacillus-Stamm das Wildtyp-Gen für die hochalkalische Protease deletiert wird, indem das besagte Wildtyp-Gen für die hochalkalische Protease durch mindestens eine Kopie der Genmutanten für eine hochalkalische Protease ersetzt wird, oder wobei in besagtem Bacillus-Stamm das Wildtyp-Gen für die hochalkalische Protease so deletiert wird, dass die im Chromosom verbleibenden Sequenzen für eine homologe Rekombination mit einem Plasmid-kodierten Protease-Gen zu kurz sind und worin mindestens eine Kopie einer Genmutanten für eine hochalkalische Protease in das Genom eingefügt oder Plasmid-kodiert ist.

8. Verfahren gemäss Anspruch 7, wobei besagter alkalophiler Bacillus-Stamm die Novo-Art PB92 von Bacillus oder einer seiner Abkömmlinge ist.

## Revendications

1. Procédé de production de protéases différant par au moins un acide aminé de la protéase de type sauvage, le procédé comprenant l'utilisation d'une souche de Bacillus alcalophile homologue comme hôte d'expression, la souche étant incapable de produire la protéase de type sauvage, où, à partir de la souche de Bacillus, le gène de la protéase très alcaline de type sauvage est délété par remplacement du gène de la protéase très alcaline de type sauvage par au moins une copie du gène mutant de la protéase très alcaline ou, où à partir de la souche de Bacillus, le gène de la protéase très alcaline de type sauvage est délété d'une telle manière que les séquences laissées dans le chromosome sont trop courtes pour une recombinaison homologue avec le gène de protéase codé par le plasmide et où au moins une copie d'un gène mutant de la protéase très alcaline est inséré dans le génome ou est codé par le plasmide.

2. Procédé suivant la revendication 1, utilisant une souche de Bacillus alcalophile protéase négative comme hôte d'expression.

3. Procédé suivant l'une quelconque des revendications 1 ou 2, utilisant un dérivé protéase négatif d'espèce nouvelle de Bacillus PB92.

4. Procédé suivant l'une quelconque des revendications 1 à 3, utilisant une souche de Bacillus alcalophile asporogène.

5. Procédé suivant l'une quelconque des revendications 1 à 4, utilisant un Bacillus asporogène à partir de laquelle le gène de protéase de type sauvage a été délété par recombinaison homologue ou illégitime.

6. Procédé suivant la revendication 1, dans lequel le gène de la protéase très alcaline de type sauvage a essentiellement la séquence en acide aminé de la protéase d'espèce nouvelle de Bacillus PB92.

7. Procédé d'obtention d'une souche de Bacillus alcalophile, capable de produire des protéases différant par au moins un acide aminé de la protéase de type sauvage, où, à partir d'une souche de Bacillus, le gène de la protéase très alcaline de type sauvage est délété par remplacement du gène de la protéase très alcaline de type sauvage par au moins une copie du gène mutant de la protéase très alcaline ou, où à partir de la souche de Bacillus, le gène de la protéase très alcaline de type sauvage est délété d'une telle manière que les séquences laissées dans le chromosome sont trop courtes pour une recombinaison homologue avec le gène de protéase codé par le plasmide et où au moins une copie d'un gène mutant de la protéase très alcaline est inséré dans le génome ou est codé par le plasmide.

8. Procédé suivant la revendication 7, dans lequel la souche de Bacillus alcalophile est une nouvelle espèce de Bacillus PB92 ou un dérivé de celle-ci.
